Europäisches Patentamt

European Patent Office

Office européen des brevets

(11) Numéro de publication: **0 177 417**
**A1**

## (12) DEMANDE DE BREVET EUROPEEN

(21) Numéro de dépôt: **85401899.1**

(22) Date de dépôt: **30.09.85**

(51) Int. Cl.⁴: **A 61 B 6/03**
**A 61 B 6/00**

(30) Priorité: **28.09.84 FR 8414926**

(43) Date de publication de la demande:
**09.04.86 Bulletin 86/15**

(84) Etats contractants désignés:
**AT BE CH DE FR GB IT LI LU NL SE**

(71) Demandeur: **Rodet, Jean-Jacques**
**19, rue du Docteur Bastié**
**F-81300 Graulhet(FR)**

(72) Inventeur: **Rodet, Jean-Jacques**
**19, rue du Docteur Bastié**
**F-81300 Graulhet(FR)**

(74) Mandataire: **Nony, Michel**
**Cabinet Nony 29, rue Cambacérès**
**F-75008 Paris(FR)**

(54) Appareil de radiologie.

(57) L'intention est relative à un appareil de radiologie, comprenant un support (1) pour le sujet, un émetter (8) de rayons X mobile autour d'un axe (1') solidaire du support (1) du sujet et une rangée (6) de détecteurs (7) de rayons X.

Ladite rangée (6) de détecteurs (7) est montée mobile par rapport à un bâti (5) pour balayer une surface solidaire de ce bâti, ledit bâti (5) étant monté sur le trajet des rayons X au-delà du support (1) du sujet de manière à se déplacer par rapport à ce support (1) en relation avec le déplacement de l'émetteur (8) de rayons X, et des moyens de mémorisation pour mémoriser les signaux de sortie des détecteurs lors du balayage effectué par la rangée (6) de détecteurs (7).

Fig.1

Appareil de radiologie.

La présente invention concerne un appareil de radiologie, et plus particulièrement un tel appareil du type comprenant un support pour le sujet et un émetteur de rayons X mobile autour d'un axe solidaire du support du sujet.

Dans ces appareils, une plaque photographique sensible aux rayons X est généralement montée de façon solidaire de l'émetteur de rayons X pour pivoter avec lui autour de l'axe, le sujet étant fixe. Des clichés sont pris sur la même plaque pour différentes positions angulaires, qui s'ajoutent les uns aux autres. Il s'agit des techniques bien connus de tomographie qui produisent sur la plaque photographique une vue en coupe du sujet, passant par l'axe de rotation.

Ces techniques de tomographie présentent toutefois un certain nombre d'inconvénients. Tout d'abord, le temps d'exposition du sujet aux rayons X est relativement important puisque le rayonnement X est émis pendant le temps que dure le balayage angulaire du couple émetteur/plaque.

Par ailleurs, les plaques photographiques sensibles aux rayons X sont onéreuses et elles ne permettent en outre que des observations relativement limitées du fait de la faible capacité de l'oeil humain de distinguer entre deux niveaux de gris différents.

Deux autres techniques de diagnostic ont été développées pour obtenir sur le plan technique de meilleurs résultats que les techniques tomographiques. Il s'agit de la tomodensitométrie axiale, ou scanner, qui est également une technique radiologique mais utilise des détecteurs de rayons X, et des procédés fondés sur le phénomène de résonance magnétique nucléaire.

Toutefois, l'utilisation de ces deux dernières techniques est limitée par le coût extrêmement élevé des matériels permettant de les mettre en oeuvre.

La présente invention vise à pallier ces inconvénients en fournissant un appareil de radiologie procurant des résultats similaires à ceux d'un tomodensitomètre mais tout en restant d'un coût raisonnable, d'autant plus qu'il peut être aisément adapté sur toute installation existante.

A cet effet, l'invention a pour objet un appareil de radiologie comprenant un support pour le sujet, un émetteur de rayons X mobile autour d'un axe solidaire du support du sujet et une rangée de détecteurs de rayons X, caractérisé par le fait que ladite rangée de détecteurs est montée mobile par rapport à un bâti pour balayer une surface solidaire de ce bâti, ledit bâti étant monté sur le trajet des rayons X au-delà du support du sujet de manière à se déplacer par rapport à ce support en relation avec le déplacement de l'émetteur de rayons X, et des moyens de mémorisation pour mémoriser les signaux de sortie des détecteurs lors du balayage effectué par la rangée de détecteurs.

L'invention se distingue donc des tomographes essentiellement par le fait qu'elle utilise, pour la réception des rayons X résiduels après la traversée du sujet, une rangée de récepteurs mobiles à la place de la plaque photographique.

Un balayage de la rangée de détecteurs pour une position donnée de l'émetteur de rayons X, et la mémorisation des signaux de sortie résultants, correspondent donc sensiblement à la prise d'un cliché en radiographie. L'ensemble des opérations de balayage et de mémorisation étant réalisé pour différentes positions angulaires de l'émetteur de rayons X et du bâti autour de l'axe solidaire du support, il est possible d'additionner numériquement les différentes images mémorisées pour obtenir une coupe tomographique parallèle à l'axe de rotation.

Mais l'appareil selon l'invention présente en outre de nombreux avantages par rapport à un appareil de tomographie.

Tout d'abord, il est possible étant donné la sensibilité et la précision des détecteurs de rayons X disponibles, d'obtenir plusieurs milliers de niveaux de gris, par exemple 4096 si on mémorise les signaux de sortie sous une forme numérique à 12 bits.

Par ailleurs, il est aisé, lors du traitement des signaux mémorisés de n'utiliser que certaines des images réalisées, ce qui permet de reconstituer des coupes correspondant à des épaisseurs choisies.

Il est également facile de soustraire une image d'une autre pour obtenir des résultats similaires à ceux de l'angiographie, ou encore, comme en tomodensitomérie axiale, de créer des "fenêtres" de niveaux de gris en limitant les différences d'amplitude des signaux, ou même, de ne pas visualiser une structure de densité déterminée en éliminant totalement certaines gammes d'amplitude des signaux.

Dans la description qui suit on envisagera uniquement le cas d'une rangée de détecteurs rectiligne effectuant son mouvement de balayage dans un plan perpendiculairement à sa direction longitudinale. Toutefois, toute autre forme de rangée de détecteurs et de surface balayée peut être envisagée, par exemple pour des raisons d'étalonnage. Toutefois, l'étalonnage peut très facilement être réalisé par des moyens électriques entre la sortie des détecteurs et la mémorisation des signaux.

Le nombre de détecteurs disposés sur la rangée, ainsi que la fréquence d'échantillonnage de la sortie de chaque détecteur lors du balayage sont choisis pour obtenir la définition d'image souhaitée. En plaçant ainsi dans la rangée un détecteur tous les 0,25 mm et en échantillonnant tous les 0,25 mm de balayage, on obtient 16 points d'image par mm pour avoir une définition similaire à celle d'une radiographie.

Dans un mode de réalisation préféré, une pièce de collimation en métal lourd est montée de façon mobile entre l'émetteur et la rangée de détecteurs de rayons X de manière à se déplacer simultanément au mouvement de balayage de cette dernière, et comporte une fente de collimation limitant le faisceau de rayons X à sa partie dirigée vers la rangée de détecteurs.

Dans ces conditions, un point donné du sujet n'est soumis au rayonnement X pour chaque image que pendant le temps très bref correspondant au passage du détecteur correspondant. L'irradiation totale du sujet est donc très inférieure à celle qui est nécessaire pour l'obtention d'une coupe tomographique.

De préférence, l'émetteur de rayons X est agencé pour pivoter sur lui-même simultanément au mouvement de balayage de la rangée de détecteurs, de manière que la même portion du faisceau de rayons X soit toujours dirigée vers la rangée de récepteurs.

Il est ainsi inutile de tenir compte lors de la mémorisation des signaux de la répartition spatiale du faisceau de rayons X issu de l'émetteur.

De même, la rangée de détecteurs est de préférence agencée pour pivoter autour de son axe longitudinal de manière que la surface de détection des détecteurs soit toujours dirigée vers la source de rayons X.

Cette disposition permet d'éviter la correction de la déviation qui se produirait dans le cas d'une incidence variable des rayons X sur les détecteurs.

Ceci peut être obtenu en prévoyant un rail pivotant autour d'un axe auquel la source de rayons X peut être solidarisée, et un chariot mobile sur ce rail, la rangée de détecteurs étant montée solidaire en rotation de ce chariot et étant guidée en translation sur ledit bâti.

On décrira maintenant à titre d'exemple non limitatif un mode de réalisation particulier de l'invention en référence aux dessins schématiques annexés dans lesquels :

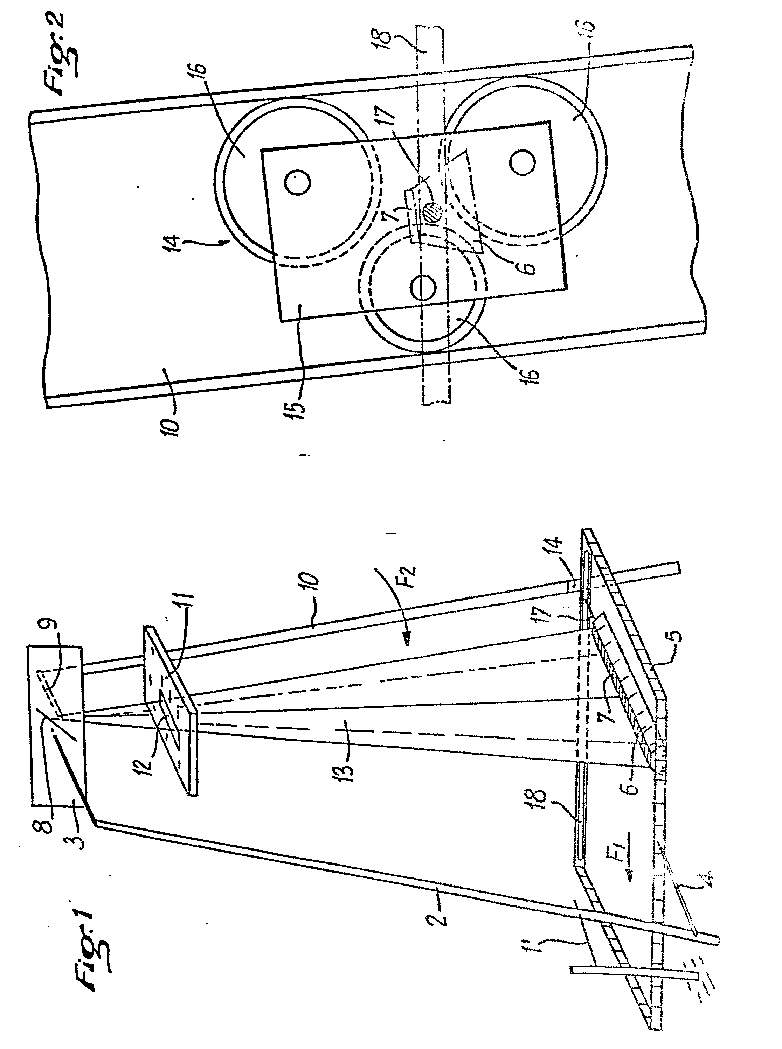

- la figure 1 est une vue en perspective d'un appareil selon l'invention,

- la figure 2 illustre un mode de réalisation particulier de la cinématique de cet appareil, et

- les figures 3a et 3b représentent le fonctionnement de cet appareil.

L'appareil représenté aux dessins comporte une table porte-malade 1 dont est solidaire un axe 1' sur lequel est monté un bras pivotant 2. Le bras 2 porte d'un côté de l'axe 1', une source 3 de rayons X de l'autre côté de l'axe 1', un axe 4 portant un bâti de support 5 pour une barrette de détecteurs rectiligne 6.

Le bâti 5 est ici constitué d'un cadre rectangulaire dont le déplacement est guidé de manière à ce qu'il reste parallèle à la table porte-malade et à l'axe 1'.

La barrette 6 porte des détecteurs 7 à semi-conducteurs, dont les sorties sont reliées à une chaîne d'acquisition de données de type connu assurant l'amplification, le filtrage, le multiplexage et la numérisation des signaux de sortie, ainsi éventuellement que leur étalonnage, préalablement à leur mémorisation, par exemple sur un disque ou une bande magnétique.

L'anode 8 de la source 3 de rayons X est montée solidaire d'un axe 9 fixé à une extrémité d'un rail 10 constitué par un profilé en U. Par conséquent, une oscillation du rail 10 autour de l'axe 9 provoque une rotation de l'anode 8, et du faisceau émis.

Une plaque de collimation 11, réalisée par exemple en plomb, comporte une fente de collimation 12 et est montée sur le rail 10 de sorte que le faisceau 13 sortant de la fente 12 soit un faisceau plan dirigée exclusivement vers les récepteurs 7 de la barrette 6.

Un chariot désigné dans son ensemble par la référence 14 est formé d'une plaque 15 sur laquelle sont montés trois galets 16 dont l'écartement est tel que deux de ces galets peuvent rouler sur une des ailes du rail 10, alors que le troisième galet roule sur l'autre aile, le chariot 14 étant à l'intérieur du rail.

La barrette 6 est montée coulissante dans le bâti 5 perpendiculairement à la direction de l'axe 1'. A cet effet, elle est supportée par une tige 17 parallèle à sa direction longitudinale et traversant à une de ses extrémités une fente de guidage 18 formée dans le bâti 5. Cette extrémité de la tige 17 est ensuite fixée à la plaque 15 du chariot 14.

Pour l'enregistrement d'une image correspondant à un angle a du bras 2 par rapport à la table 1, la barrette 6 est placée à une extrémité du bâti 5, puis elle est déplacée dans le sens de la flèche F1 de manière à balayer l'espace compris dans le bâti 5. Pendant ce mouvement de balayage, la tige 17 coulisse dans la fente 18 et entraîne simultanément le rail 10 dans un mouvement de pivotement F2 autour de l'axe 9.

Du fait que les galets 16 maintiennent constante l'orientation de la plaquette 15 par rapport au rail 10, les détecteurs 7 restent en permanence orientés vers l'anode 8 puisque la tige 17 est solidaire de la plaque 15.

TG/JD/AO/CDE72027

0177417

L'image est entièrement enregistrée lorsque la barrette 6 et le rail 10 sont arrivés dans leur position représentée en traits mixtes sur la figure 3a. On constate que, pendant ce mouvement, la plaque de collimation 11 et l'anode 8 ont suivi le mouvement de rotation autour de l'axe 9.

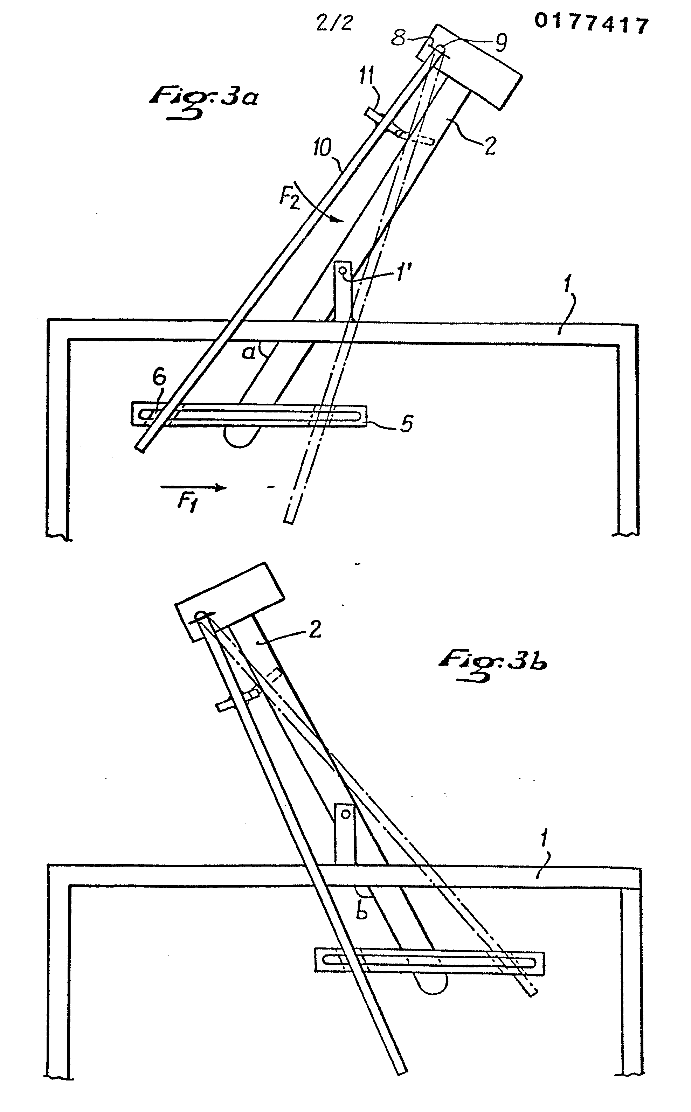

D'autres images peuvent alors être enregistrées avec d'autres valeurs b de l'angle entre le bras 2 et la table 1, comme cela est représenté à la figure 3b. Les images enregistrées pour les différentes valeurs de cet angle peuvent ensuite être traitées comme cela a été exposé ci-dessus.

Diverses variantes et modifications peuvent bien entendu être apportées à la description ci-dessus sans sortir pour autant du cadre ni de l'esprit de l'invention.

## REVENDICATIONS

1. Appareil de radiologie, comprenant un support (1) pour le sujet, un émetteur (8) de rayons X mobile autour d'un axe (1') solidaire du support du sujet et une rangée de détecteurs de rayons X, caractérisé par le fait que ladite rangée (6) de détecteurs (7) est montée mobile par rapport à un bâti (5) pour balayer une surface solidaire de ce bâti, ledit bâti étant monté sur le trajet des rayons X au-delà du support du sujet de manière à se déplacer par rapport à ce support en relation avec le déplacement de l'émetteur de rayons X, et des moyens de mémorisation pour mémoriser les signaux de sortie des détecteurs lors du balayage effectué par la rangée de détecteurs.

2. Appareil selon la revendication 1, caractérisé par le fait qu'une pièce de collimation (11) en métal lourd est montée de façon mobile entre l'émetteur et la rangée de détecteurs de rayons X, de manière à se déplacer simultanément au mouvement de balayage de cette dernière, et comporte une fente de collimation (12) limitant le faisceau de rayons X à sa partie dirigée vers la rangée de détecteurs.

3. Appareil selon l'une quelconque des revendications 1 et 2, caractérisé par le fait que l'émetteur de rayons X est agencé pour pivoter sur lui-même simultanément au mouvement de balayage de la rangée de détecteurs de manière que la même portion du faisceau de rayons X soit toujours dirigée vers la rangée de récepteurs.

4. Appareil selon l'une quelconque des revendications 1 à 3, caractérisé par le fait que la rangée de détecteurs est agencée pour pivoter autour de son axe longitudinal de manière que la surface de détection des détecteurs soit toujours dirigée vers la source de rayons X.

5. Appareil selon l'une quelconque des revendications 1 à 4, caractérisé par le fait qu'il comporte un rail (10) pivotant autour d'un axe (9) auquel la source de rayons X peut être solidarisée, et un chariot (14) mobile sur ce rail, la rangée de détecteurs étant montée solidaire en rotation de ce chariot et étant guidée en translation sur ledit bâti.

TG/JD/AO/CDE72027

Fig.1

Fig.2

1/2

Fig. 3a

Fig. 3b

**0177417**

Office européen
des brevets

**RAPPORT DE RECHERCHE EUROPEENNE**

Numero de la demande

EP 85 40 1899

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| Y | GB-A-2 133 246 (PICKER INTERNATIONAL LTD.) * Figures 1,2; page 1, ligne 59 - page 2, ligne 65 * | 1,3 | A 61 B 6/03 A 61 B 6/00 |
| A | | 2 | |
| Y | US-A-3 733 487 (LOUCHE et al.) * Figures 1,2; colonne 2, ligne 53 - colonne 3, ligne 42 * | 1,3 | |
| A | | 5 | |
| A | US-A-4 179 100 (SASHIN) * Figures 1a,b,6a,b,10,11; colonne 4, ligne 43 - colonne 5, ligne 47; colonne 9, lignes 26-59; colonne 11, ligne 13 - colonne 12, ligne 9 * | 1,2,3 | DOMAINES TECHNIQUES RECHERCHES (Int. Cl.4) A 61 B |
| A | US-A-4 158 777 (HOGAN) * Abrégé; figures 3,8; colonne 4, ligne 58 - colonne 5, ligne 7 * | 1,3,4 | |

Le présent rapport de recherche a été établi pour toutes les revendications

| Lieu de la recherche | Date d'achèvement de la recherche | Examinateur |
|---|---|---|
| LA HAYE | 04-12-1985 | CHEN A.H. |